# EUROPEAN PATENT APPLICATION

(11) **EP 4 000 617 A1**
(43) Date of publication of application: **25.05.2022**
(21) Application number: 20383021.1
(22) Date of filing: 23.11.2020
(51) Int. Cl.: A61K 31/381, A61K 31/4535, A61K 31/496, A61P 35/02

(54) **COMPOSITIONS FOR USE IN THE TREATMENT OF LEUKAEMIA**

(71) Applicant: Fundació Institut de Recerca Contra la Leucèmia Josep Carreras, 08021 Barcelona (ES)
(72) Inventor: MUÑOZ RISUEÑO, Ruth, 08021 Barcelona (ES); CORNET MASANA, Josep Maria, 08021 Barcelona (ES); CARBÓ MARQUÉS, José María, 08021 Barcelona (ES)
(74) Representative: Hoffmann Eitle

(57) **Abstract**

The present invention relates to a method of treating a warm-blooded animal, especially a human subject, having leukaemia, preferably acute myeloid leukemia (AML), B and T acute lymphoblastic leukemias (B-ALL and T-ALL), or chronic myeloid leukemia (CML), or a lymphoma or a solid tumour, comprising administering to said animal a therapeutically effective amount of a compound having any of the formulae identified throughout the present specification, optionally together or in combination with a conventional compound or compound mixture useful in any of said treatments, in particular a topoisomerase II inhibitor, an antimetabolite, or an antitumor antibiotic, and optionally at least one pharmaceutically acceptable carrier, for simultaneous, separate or sequential use; and to a pharmaceutical composition and a commercial package comprising said compound.

## Description

### TECHNICAL FIELD

The present invention relates to a method of treating a warm-blooded animal having leukaemia, preferably acute myeloid leukaemia (AML), B and T acute lymphoblastic leukemias (B-ALL and T-ALL), and chronic myeloid leukaemia (CML), as well as lymphoma and solid tumours, comprising administering to said animal a therapeutically effective amount of a compound having any of the formulae identified throughout the present specification, optionally together or in combination with a conventional compound or compound mixture useful in said treatment, and to a pharmaceutical composition and a commercial package comprising said compound.

### BACKGROUND OF THE INVENTION

Leukemia, also spelled leukaemia, is a group of blood cancers that usually begin in the bone marrow and result in high numbers of abnormal blood cells. These blood cells are not fully developed and are called blasts or leukaemia cells. Symptoms may include bleeding and bruising, feeling tired, fever, and an increased risk of infections. These symptoms occur due to a lack of normal blood cells. Diagnosis is typically made by blood tests or bone marrow biopsy.

The exact cause of leukaemia is unknown. A combination of genetic factors and environmental (non-inherited) factors are believed to play a role. Risk factors include smoking, ionizing radiation, some chemicals (such as benzene), prior chemotherapy, and Down syndrome. People with a family history of leukaemia are also at higher risk. There are four main types of leukaemia-acute lymphoblastic leukaemia (ALL), acute myeloid leukaemia (AML), chronic lymphocytic leukaemia (CLL) and chronic myeloid leukaemia (CML)-as well as a number of less common types. Leukemias and lymphomas both belong to a broader group of tumours that affect the blood, bone marrow, and lymphoid system, known as tumours of the hematopoietic and lymphoid tissues.

Treatment may involve some combination of chemotherapy, radiation therapy, targeted therapy, and bone marrow transplant, in addition to supportive care and palliative care as needed. Certain types of leukaemia may be managed with watchful waiting. The success of treatment depends on the type of leukaemia and the age of the person. In this sense, although outcomes have improved in the developed world, there is still a need to find alternative treatments that improved the success of treatment.

### BRIEF DESCRIPTION OF THE FIGURES

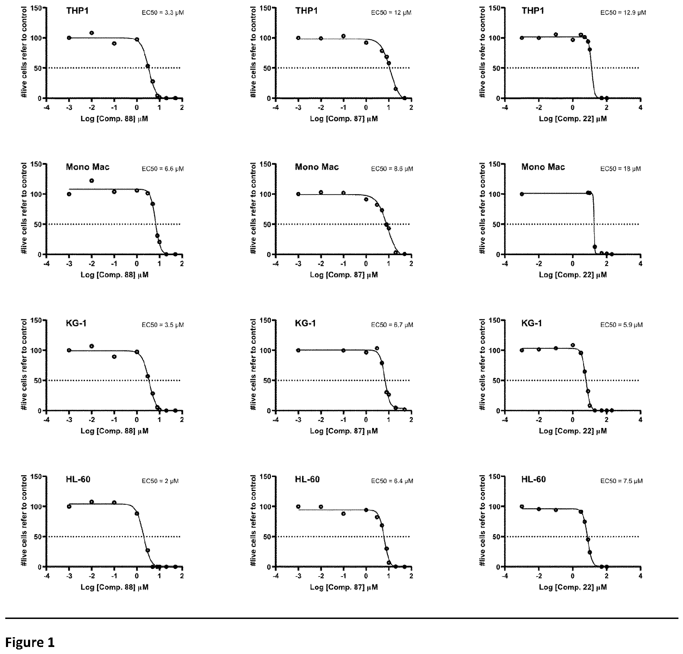

**Figure 1****.** Four different AML cell lines (THP-1, MonoMac-1, KG-1, HL-60) were cultured for 72 h in complete RPMI. Compounds 87, 88 and 22 were added at different concentrations. Cell viability was measured by flow cytometry analysis (FSC-SSC profile, 7-AAD negativity, Hoechst 344442 dim, volumetric count). Cell viability, related to vehicle-control treated samples, is represented. EC50 value was measured using GraphPad Prism software.

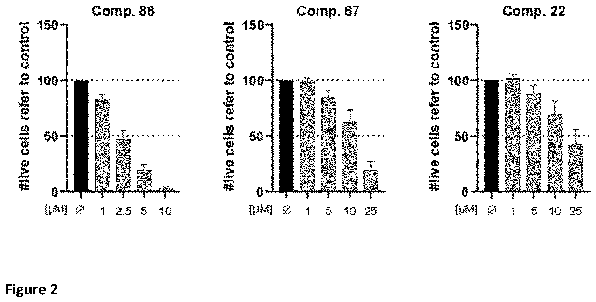

**Figure 2****.** Four different AML cell lines (THP-1, MonoMac-1, KG-1, HL-60) were cultured for 72 h in complete RPMI. Compounds 87, 88 and 22 were added at 1/20.5/5/10 µM. Cell viability was measured by flow cytometry analysis (FSC-SSC profile, 7-AAD negativity, Hoechst 344442 dim, volumetric count). Cell viability, related to vehicle-control treated samples, is represented. Results from all cell lines were pulled and averaged.

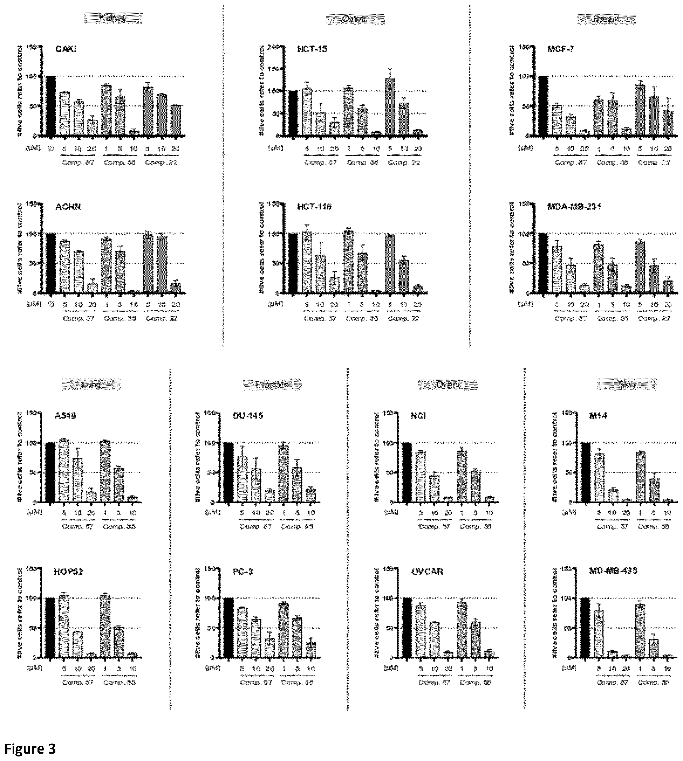

**Figure 3****.** Each indicated solid tumor (kidney, colon, breast, lung, prostate, ovary and skin cancer) cell line was culture for 72 h in complete RPMI. Compound 87, 88 and 22 were added at 1/5/10/20 µM. Cell viability was measured by metabolic analysis, using the CellTiter-Flour^{™} Cell Viability Assay, following the manufactures' recommendations. Cell viability, related to vehicle-control samples, is represented.

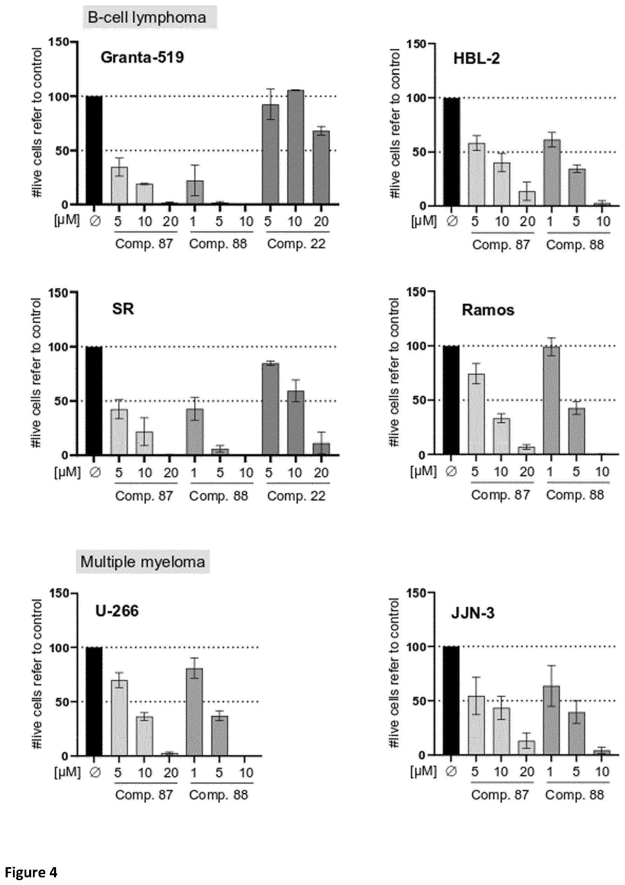

**Figure 4****.** Each indicated B-cell lymphoma and multiple myeloma cell line was culture for 72 h in complete RPMI. Compound 87, 88 and 22 were added at 1/5/10/20 µM. Cell viability was measured by flow cytometry analysis (FSC-SSC profile, 7-AAD negativity, Hoechst 344442 dim, volumetric count). Cell viability, related to vehicle-control samples, is represented.

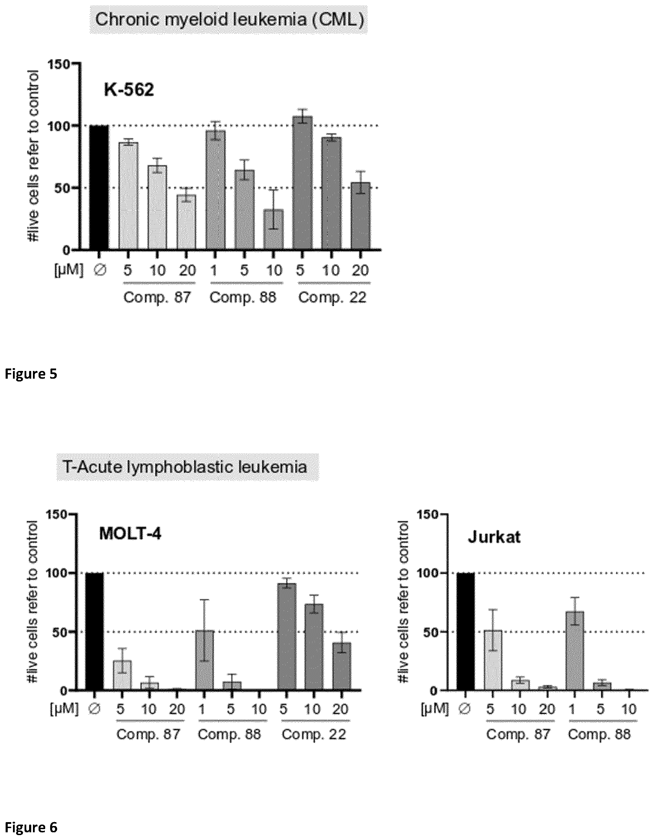

**Figure 5****.** The chronic myeloid leukemia cell line was culture for 72 h in complete RPMI. Compound 87, 88 and 22 were added at 1/5/10/20 µM. Cell viability was measured by flow cytometry analysis (FSC-SSC profile, 7-AAD negativity, Hoechst 344442 dim, volumetric count). Cell viability, related to vehicle-control samples, is represented.
**Figure 6****.** Each indicated T-cell acute lymphoblastic cell line was culture for 72 h in complete RPMI. Compound 87, 88 and 22 were added at 1/5/10/20 µM. Cell viability was measured by flow cytometry analysis (FSC-SSC profile, 7-AAD negativity, Hoechst 344442 dim, volumetric count). Cell viability, related to vehicle-control samples, is represented.

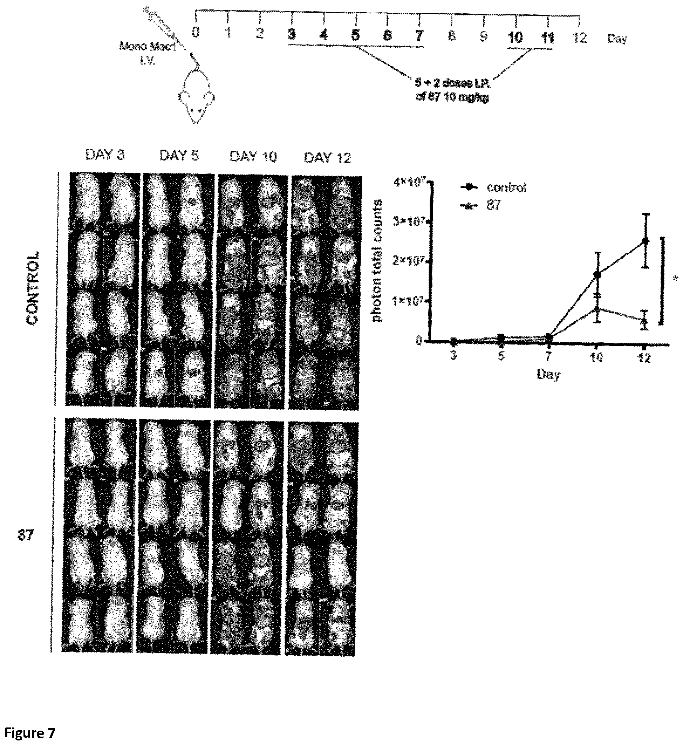

**Figure 7****.** Six to ten-week-old adult NOD-scid IL2Rgamma null (NSG) mice were myelo-ablationed with 30 mg/kg busulfan (intraperitoneal administration). 1 x 10 6 Luc-positive MonoMac-1 AML cells were transplanted in the tail vain 24 h after conditioning. Mice were treated on days 3, 4, 5, 6, 7, 10 and 11 with compound 87 (10 mg/kg), intraperitonally. On day 3, 5, 10 and 12, mice were longitudinally imaged after D-luciferin injection, to detect AML cells. Serial images of 4 representative mice at different time points are shown. Bioluminescence imaging was quantified and expressed as luminescent intensity (photon total counts), as a measured of leukemic cell burden.

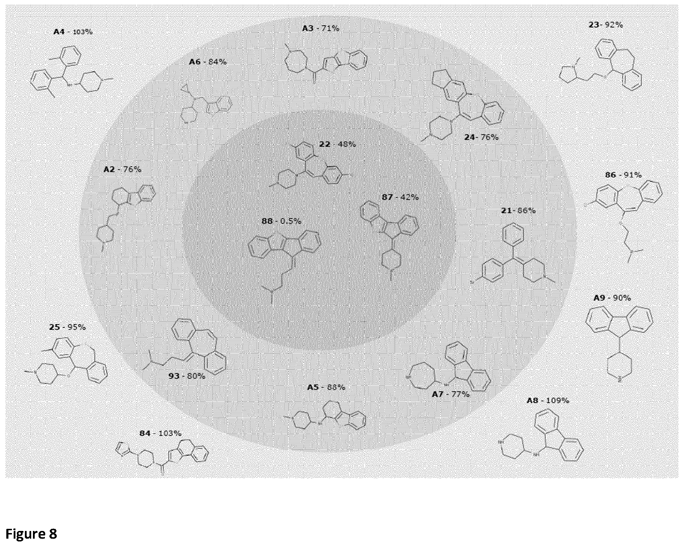

**Figure 8****.** THP-1, HL-60, MonoMac-1 and KG-1 AML cells were treated with 10 µM of each indicated compound for 48h. Cell viability was measured by flow cytometry (FSC-SSC profile, 7-AAD negative, Hoechst 33342 dim), using a volumetric count. The frequency of live cells compared to vehicle control-treated samples is expressed as the average value of each cell line, run in technical triplicates twice. Compounds are represented based on their efficiency in inducing cell death. As shown in figure 8, only compounds 22, 87, and 88 reduced the cell viability of AML cells to 48, 42, and 0.5 respectively.

### DESCRIPTION OF THE INVENTION

### Definitions

The term "treatment" as used herein comprises the treatment of patients having leukaemia, preferably acute myeloid leukaemia (AML), B and T acute lymphoblastic leukemias (B-ALL and T-ALL), or chronic myeloid leukaemia (CML), or a lymphoma or a solid tumour, or being in a pre-stage or a post-remission stage of any of these diseases. The treatment effects the delay of progression or the partial or complete elimination of the disease in said patients.

The term "leukaemia" as used herein relates to a malignant cancer of bone marrow and blood, characterized by the uncontrolled growth of blood cells. Leukaemia can be further subdivide based on the cellular lineage (myeloid and lymphoid) and natural history (acute and chronic).

The term "B and T acute lymphoblastic leukemias (B-ALL and T-ALL)" refers to a malignant transformation of the lymphoid cells that results in the uncontrolled proliferation of B and T cells, that accumulate in bone marrow and other peripheral organs. It progresses quickly and aggressively, requiring immediate treatment. B-ALL and T-ALL show an increase incident rate in paediatric patients, as compared to adults. Currently, refractoriness and relapses are the main clinic challenges.

The term "chronic myeloid leukaemia (CML)" refers to a myeloproliferative disorder characterized by increase proliferation of transformed granulocytes, lacking the capacity to differentiate.

The term "lymphoma" refers to group of neoplasms originated from transformed lymphocytes that affect the lymph system. There are two main types of lymphoma: Hodgkin lymphoma and non-Hodgkin lymphoma, depending on cellular characteristics and spreading facility.

The term "solid tumour" refers to a pathological state where transformed cells are accumulated into discrete solid masses. Solid tumors include cancers of kidney, colon, breast, lung, prostate, ovary, skin and other tissues.

The term "acute myeloid leukaemia" as used herein relates to an uncontrolled, quickly progressing growth of myeloid cells, e.g. granulocytes, as well as erythroid and megakaryotic cells and progenitors. In patients with AML the immature myeloid, erythroid or megakaryotic cells severely outnumber erythrocytes (red blood cells) leading to fatigue and bleeding, and also to increased susceptibility to infection. In children as well as in adults AML has a poor prognosis despite the use of aggressive chemotherapeutic protocols. Overall survival rates are 40-60%. Autologous bone marrow transplant preceded by myeloablative chemotherapy does not change the survival, but an allogeneic bone marrow transplant preceded by aggressive chemotherapy might increase the survival rates up to 70%. Unfortunately, the availability of a matched sibling donor is limited. Therefore, new therapeutic strategies in AML treatment are necessary.

### Description

As shown in the figures and in the examples of the present specification, THP-1, HL-60, MonoMac-1 and KG-1 AML cells treated with 10 µM of each of the compounds indicated in figure 8 for 48h, yielded that only compounds 22, 87, and 88 reduced the cell viability of AML cells to 48, 42, and 0.5% respectively (see figure 8). These 3 compounds were then assayed as illustrated in figures 1 to 6 demonstrating an *in vitro* anti-leukemic activity. Also, as shown in figure 7, mice intravenously transplanted with 1 x 10⁶ Luc-positive MonoMac-1 AML cells and treated on days 3, 4, 5, 6, 7, 10 and 11 with compound 87 (10 mg/kg), intraperitoneally, detected a remarkable reduction in the amount of AML cells at day 12 in comparison to the control.

The present invention thus relates to a method of treating a warm-blooded animal, especially a human subject, having leukaemia, preferably acute myeloid leukemia (AML), B and T acute lymphoblastic leukemias (B-ALL and T-ALL) or chronic myeloid leukemia (CML), or a lymphoma or a solid tumour, comprising administering to said animal a therapeutically effective amount of a compound having any of the formulae identified throughout the present specification, optionally together or in combination, for simultaneous, separate or sequential use, with a conventional compound or compound mixture useful in any of said treatments, in particular a topoisomerase II inhibitor, an antimetabolite, or an antitumor antibiotic, and optionally at least one pharmaceutically acceptable carrier. The present invention further relates to pharmaceutical compositions and a commercial package comprising said compounds.

Hence, a first aspect of the invention relates to a method of treating leukaemia, preferably acute myeloid leukemia (AML), B and T acute lymphoblastic leukemias (B-ALL and T-ALL), or chronic myeloid leukemia (CML), or a lymphoma or a solid tumour, comprising administering to a warm-blooded animal, preferably a human in need thereof, a therapeutically effective amount of a compound, or a salt thereof, selected from those comprising or consisting of formula I: 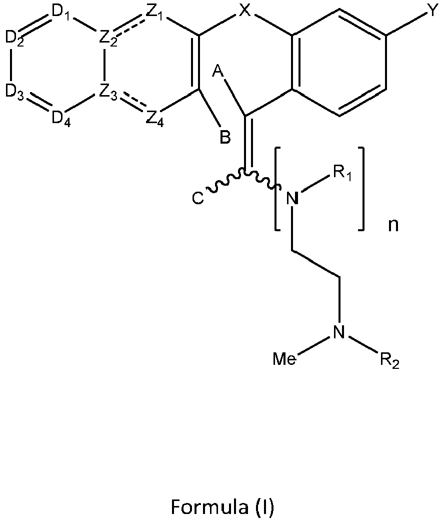 wherein when n = 0:
- A and B together represent a single bond forming a 5 or 6 membered ring;
- Y is a hydrogen atom;
- X can be independently selected from a sulphur atom or represents a single bond so as to form a 5 membered ring, wherein:
   ∘ when X is a sulphur atom forming a 6 membered ring:
      ▪ R2 is a methyl group and C is a hydrogen atom;
      ▪ Z1, Z2 and Z4 are CH groups
      ▪ Z3 is a carbon atom, D4 is a Chlorine atom; and
      ▪ D1, D2 and D3 are absent
   ∘ when X is a single bond forming a 5 membered ring:
      ▪ R2 is a methyl or methylene group;
      ▪ C can be selected from a hydrogen atom or a methylene group; wherein when C is a methylene group, R2 is a methylene linked to C forming a 6 membered N methyl-piperidine;
      ▪ Z1, Z4 are both independently selected from a sulphur atom or a single bond forming a 5 membered ring; so that when Z1 is a sulphur atom, Z4 forms a single bond forming a 5 membered ring; or when Z4 is a sulphur atom, Z1 forms a single bond forming a 5 membered ring;
      ▪ Z2 and Z3 are both carbon atoms; and
      ▪ D1-D4 are carbon atoms;
         and
wherein, when n is 1:
- X is a sulphur atom;
- B and C together represent a single bond forming a 7 membered sulphur containing heterocyclic ring;
- A is absent and both R1 and R2 are both methylene groups linked together so as to form a 6 membered heterocyclic ring;
- Y is a Chlorine atom;
- Z1, Z2 and Z4 are CH groups; and
- Z3 is a carbon atom, D1 is H, D2 and D3 are absent, and D4 is a fluorine atom.

It is noted that the following bond exemplified below from Formula I: 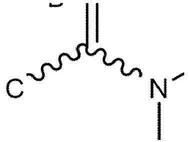 shall be understood as encompassing isomers.

Preferably, the treatment indicated in the first aspect of the invention is preferably administered together or in combination with a conventional compound useful in a method of treating leukaemia, preferably acute myeloid leukemia (AML), B and T acute lymphoblastic leukemias (B-ALL and T-ALL), or chronic myeloid leukemia (CML), more preferably acute myeloid leukaemia.

In a preferred embodiment of the invention the compound, including isomers and salts thereof, of formula I is selected from any of formulae:
- 1-methyl 4-(6H-benz[b]indeno[1,2-d]thien-6-ylidene) piperidine (88);
- N,N-dimethyl 3-(10H-benz[b]indeno[2,1-d]thien-10-ylidene) -1-Propanamine (87); and
- 1-methyl -4-(8-chloro-3-fluorodibenzo[b,f]thiepin-10-yl)- Piperidine(22).

The chemical formulae for each of these compounds is represented herein below:

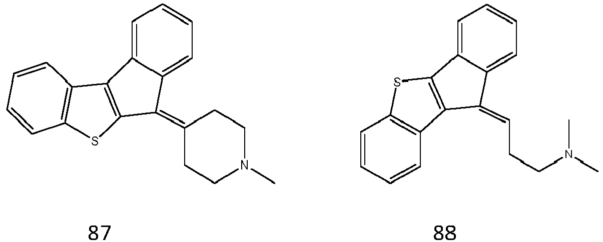

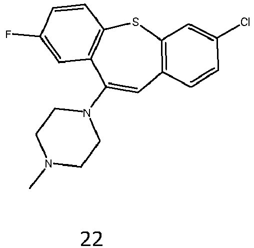

In a further preferred embodiment of the invention, the compound, including isomers and salts thereof, of formula I is:

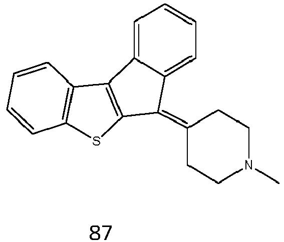

It will be understood that throughout the present specification, references to the active ingredients or **"compounds of the invention"** are meant to refer to the above compounds of formulae I and to include pharmaceutically acceptable salts and isomers thereof. If these active ingredients have, for example, at least one basic centre, they can form acid addition salts. The active ingredient or a pharmaceutically acceptable salt thereof may also be used in form of a hydrate or include other solvents used for crystallization.

It is noted that, preferably, any of the treatment methods indicated in any of the previous embodiments, is preferably administered together or in combination with a conventional compound useful in a method of treating leukaemia, preferably acute myeloid leukemia (AML), B and T acute lymphoblastic leukemias (B-ALL and T-ALL) or chronic myeloid leukemia (CML). More preferably, the method of treatment is for treating acute myeloid leukaemia.

Conventional compounds useful in the treatment of leukaemia, preferably acute myeloid leukemia (AML) comprise, but are not limited to, topoisomerase II inhibitors, such as amsacrine, etoposide or teniposide, anitmetabolites, such as cytarabine, methotexate or mercaptopurine, or antitumor antibiotics, such as mitoxantrone, dactinomycin, daunorubicin, doxorubicin, epirubicin, homoharringtonine or idarubicin. Other conventional compounds useful in the treatment of leukaemia considered in this invention are compounds usually applied in the treatment of ALL (acute lymphoblastic leukemia), such as asparaginase, cyclophosphamide, gemtuzumab (or any other CD 33 monoclonal antibody), ifosfamide, mesna, prednisone, topotecan, and vincristine. Under the term "conventional compound" also mixtures of the mentioned compounds are understood, e.g. combinations of cytarabine with mitoxantrone, amsacrine, daunorubicin, etoposide or idarubicin, or of cytarabine with etoposide and daunorubicin or mitoxantrone.

Preferred conventional compounds used in the invention are amsacrine, etoposide, cytarabine, daunorubicin and mitoxantrone, and mixtures thereof, in particular amsacrine, cytarabine and mitoxantrone. Particularly preferred are conventional compounds for paediatric use.

It is herein understood, that any of the methods of the invention might be characterized by a multiple treatment regimen, such as a triple or quadruple therapy, comprising administering a compound of the invention together or in combination with one, two or three conventional compounds useful in the treatment of leukaemia, preferably acute myeloid leukemia (AML), B and T acute lymphoblastic leukemias (B-ALL and T-ALL), or chronic myeloid leukemia (CML), more preferably acute myeloid leukaemia.

Particularly preferred is the treatment according to the invention when applied to juveniles. Hence, another preferred embodiment of the invention, relates to the administration of any of the treatment methods indicated in any of the previous aspects of the invention or in any of its preferred embodiments to a juvenile having leukemia, preferably acute myeloid leukemia (AML), B and T acute lymphoblastic leukemias (B-ALL and T-ALL), or chronic myeloid leukemia (CML), more preferably acute myeloid leukaemia, which is optionally resistant to conventional chemotherapy, comprising administering to the juvenile human in need thereof a therapeutically effective amount of a compound of the invention as defined hereinbefore, in particular by administering any of compounds 87, 88 and/or 22 or any pharmaceutical acceptable salt or isomer thereof. Preferably, said treatment is preferably administered together or in combination with a conventional compound useful in a method of treating leukaemia, preferably acute myeloid leukemia (AML), B and T acute lymphoblastic leukemias (B-ALL and T-ALL), or chronic myeloid leukemia (CML), more preferably acute myeloid leukaemia.

The method of administering a compound of the invention as defined hereinbefore may further comprise one or more pharmaceutically acceptable carriers, and may optionally involve simultaneous, separate or sequential application of conventional compounds.

In a further aspect the invention relates to combined pharmaceutical preparations, especially a "kit of parts" in the sense that the conventional compounds and the compounds of the invention as defined above can be dosed independently or by use of different fixed combinations with distinguished amounts of the ingredients, i.e., simultaneously or at different time points. The parts of the kit can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the active ingredients. The ratio of the total amounts of the compound of the invention as defined hereinbefore to the active ingredient(s) of the conventional compound useful in the treatment of leukaemia, such as AML, to be administered in the combined preparation can be varied, e.g., in order to cope with the needs of a patient sub-population to be treated or the needs of the single patient, which different needs can be due to age, sex, body weight, etc. of the patients. Especially preferred is an administration regime taking into account special needs of paediatric use. Preferably, there is at least one beneficial effect, e.g., a mutual enhancing of the effect of the compound of the invention as defined hereinbefore and of the conventional compound, in particular a synergism, e.g. a more than additive effect, additional advantageous effects, less side effects, a combined therapeutically effect in a non-effective dosage of one or each of the active ingredients, and especially a strong synergism of the a compound of the invention as defined hereinbefore and the conventional compound useful in the treatment of leukaemia, preferably acute myeloid leukemia (AML), B and T acute lymphoblastic leukemias (B-ALL and T-ALL), or chronic myeloid leukemia (CML), more preferably acute myeloid leukaemia.

The person skilled in the pertinent art is fully enabled to select relevant test models to prove the hereinbefore and hereinafter mentioned beneficial effects on treatment of leukaemia, preferably acute myeloid leukemia (AML), B and T acute lymphoblastic leukemias (B-ALL and T-ALL), or chronic myeloid leukemia (CML), more preferably acute myeloid leukaemia, of a compound of the invention as defined hereinbefore alone or together or in combination with a conventional compound useful in the treatment of any of these diseases. The activity of the single compounds of the invention or of a combination of the invention may, for example, be demonstrated in a suitable clinical study. Suitable clinical studies are, for example, open label non-randomized, dose escalation studies in patients with advanced AML, preferably in juvenile patients. Such studies prove in particular the synergism observed with a compound of the invention as defined hereinbefore or with the combinations of the invention. The beneficial effects on treatment of leukaemia, preferably acute myeloid leukemia (AML), B and T acute lymphoblastic leukemias (B-ALL and T-ALL), or chronic myeloid leukemia (CML), more preferably acute myeloid leukaemia, can be determined directly through the results of such studies or by changes in the study design which are known as such to a person skilled in the art. For example, one combination partner can be administered with a fixed dose and the dose of a second combination partner is escalated until the Maximum Tolerated Dosage (MTD) is reached. Alternatively, a placebo-controlled, double blind study can be conducted in order to prove the benefits of the combination of the invention mentioned herein.

In a further aspect the invention also concerns pharmaceutical compositions. The pharmaceutical compositions for sole administration of a compound of the invention as defined hereinbefore, or for separate administration of the combination partners or for the administration in a fixed combination, i.e. a single galenical composition comprising at least two combination partners, according to the invention can be prepared in a manner known per se and are those suitable for enteral, such as oral or rectal, and parenteral administration to warm-blooded animals, including man, comprising a therapeutically effective amount of at least one pharmacologically active compound of the invention as defined hereinbefore, or a combination partner alone or in combination with one or more pharmaceutically acceptable carries, especially suitable for enteral or parenteral application.

Novel pharmaceutical compositions contain, for example, from about 10% to about 100%, preferably from about 20% to about 60%, of the active ingredients or compounds of the invention as defined hereinbefore. Pharmaceutical preparations to carry out the present invention for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the combination of the invention may be administered simultaneously or sequentially and, in any order, and the components may be administered separately or as a fixed combination. For example, the method of treatment of leukaemia, preferably acute myeloid leukemia (AML), B and T acute lymphoblastic leukemias (B-ALL and T-ALL), or chronic myeloid leukemia (CML), more preferably acute myeloid leukaemia, according to the present invention may comprise (i) administration of a first combination partner in free or pharmaceutically acceptable salt form, (ii) administration of a second combination partner in free or pharmaceutically acceptable salt form, and, optionally (iii) administration of a third, forth, etc. combination partner in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the combination of the invention can be administered separately at different times during the course of therapy or concurrently in divided or single combination forms. Furthermore, the term administering also encompasses the use of a pro-drug of a combination partner that convert in vivo to the combination partner as such. The instant invention is therefore to be understood as embracing all such regimes of simultaneous or alternating treatment and the term "administering" is to be interpreted accordingly.

The effective dosage of the compounds of the invention as defined hereinbefore and of the combination partners employed in the combination of the invention may vary depending on the particular compound or pharmaceutical composition employed, the mode of administration, the stage of progression of the leukaemia, preferably acute myeloid leukemia (AML), B and T acute lymphoblastic leukemias (B-ALL and T-ALL), or chronic myeloid leukemia (CML), more preferably acute myeloid leukaemia, being treated, the severity of the leukaemia, preferably acute myeloid leukemia (AML), B and T acute lymphoblastic leukemias (B-ALL and T-ALL), or chronic myeloid leukemia (CML), more preferably acute myeloid leukaemia, being treated, and the responsiveness of the patient being treated. Thus, the dosage regimen of the combination of the invention is selected in accordance with a variety of factors including the route of administration and the renal and hepatic function of the patient. A physician, clinician or veterinarian of ordinary skill can readily determine and prescribe the effective amount of a compound of formula I or of the other single active ingredients of the combination of the invention required to prevent, counter or arrest the progress of the condition. Optimal precision in achieving concentration of the active ingredients within the range that yields efficacy without toxicity requires a regimen based on the kinetics of the active ingredients' availability to target sites.

If the warm-blooded animal is an adult human, the dosage of a compound of the invention as defined hereinbefore, especially compounds 87, is preferably in the range of about 20 to 1500, more preferably about 20 to 100, and most preferably 50 to 100 mg/day. In the combination of the invention, the dosage of a compound of the invention as defined hereinbefore may be reduced compared to single application. The dosage of compound 87, for example, in a combination or together with a conventional compound useful in the treatment of AML is preferably in the range of 20 to 1000, more preferably about 50 to 500, and most preferably 50 to 100 mg/day. The preferred dosage of the other combination partner, i.e. the conventional compound for treatment of AML, is also reduced accordingly, preferably to around 50% of the standard dosage.

For use with children, the dosage is reduced accordingly, and adapted to the body weight and/or surface area of the juvenile.

The following examples merely illustrate the present invention but do not limit the same.

### EXAMPLES

### Example 1. Efficiency in inducing cell death of the compounds represented in figure 8.

0.15 x 106 AML cell lines (THP-1, KG-1, HL-60 or MonoMac-1) per mL were cultured in completed RPMI medium, supplemented with 10% FBS. Compounds were added to the medium at 10 µM), while controls were treated with equivalent concentration of vehicle (DMSO). Primary validation screenings were analyzed after 48 h of treatment. Cells were stained with the live-dead discrimination dye 7-AAD and Hoechst 33442, and acquired in a flow cytometer, with a volumetric counting. Live cells were discriminated based on a discrete FSC-SSC profile, 7-AAD negativity and dim positivity to Hoechst 34442.

The results are illustrated in figure 8.

### Example 2. Mice intravenously transplanted with 1 x 106 Luc-positive MonoMac-1 AML cells treated with compound 87.

Six to ten-week-old adult NOD-*scid* IL2Rgamma^{null} (NSG) mice were myelo-ablationed with 30 mg/kg busulfan (intraperitoneal administration). 1 x 10⁶ Luc-positive MonoMac-1 AML cells were transplanted in the tail vain 24 h after conditioning. Mice were treated on days 3, 4, 5, 6, 7, 10 and 11 with compound 87 (10 mg/kg), intraperitonally. On day 3, 5, 10 and 12, mice were longitudinally imaged after D-luciferin injection, to detect AML cells. Serial images of 4 representative mice at different time points are shown. Bioluminescence imaging was quantified and expressed as luminescent intensity (photon total counts), as a measured of leukemic cell burden.

The results are illustrated in figure 7.

## Claims

1. A composition for use in a method of treating leukaemia comprising administering to a warm-blooded animal in need thereof a therapeutically effective amount of a compound, or a salt thereof, selected from those comprising formula I: 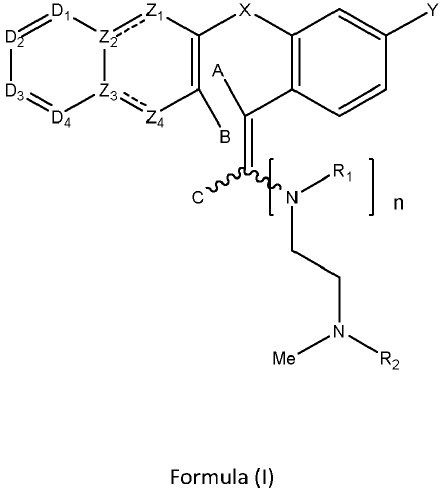 wherein when n = 0:
- A and B together represent a single bond forming a 5 or 6 membered ring;
- Y is a hydrogen atom;
- X can be independently selected from a sulphur atom or represents a single bond so as to form a 5 membered ring, wherein:
∘ when X is a sulphur atom forming a 6 membered ring:
▪ R2 is a methyl group and C is a hydrogen atom;
▪ Z1, Z2 and Z4 are CH groups
▪ Z3 is a carbon atom, D4 is a Chlorine atom; and
▪ D1, D2 and D3 are absent
∘ when X is a single bond forming a 5 membered ring:
▪ R2 is a methyl or methylene group;
▪ C can be selected from a hydrogen atom or a methylene group; wherein when C is a methylene group, R2 is a methylene linked to C forming a 6 membered N methyl-piperidine;
▪ Z1, Z4 are both independently selected from a sulphur atom or a single bond forming a 5 membered ring; so that when Z1 is a sulphur atom, Z4 forms a single bond forming a 5 membered ring; or when Z4 is a sulphur atom, Z1 forms a single bond forming a 5 membered ring;
▪ Z2 and Z3 are both carbon atoms; and
▪ D1-D4 are carbon atoms;
and
wherein, when n is 1:
- X is a sulphur atom;
- B and C together represent a single bond forming a 7 membered sulphur containing heterocyclic ring;
- A is absent and both R1 and R2 are both methylene groups linked together so as to form a 6 membered heterocyclic ring;
- Y is a Chlorine atom;
- Z1, Z2 and Z4 are CH groups;
- Z3 is a carbon atom, D1 is H, D2 and D3 are absent, and D4 is a fluorine atom.

2. The composition for use according to claim 1, wherein n = 0, A and B together represent a single bond forming a 5 or 6 membered ring, Y is a hydrogen atom, and X can be independently selected from a sulphur atom or represents a single bond forming a 5 membered ring, wherein:
when X is a sulphur atom a 6 membered ring is formed, wherein:
R2 is a methyl group and C is a hydrogen atom;
Z1, Z2 and Z4 are CH groups;
Z3 is a carbon atom, D4 is a Chlorine atom; and
D1, D2 and D3 are absent
when X is a single bond a 5 membered ring is formed, wherein:
R2 is a methyl or methylene group;
C can be selected from a hydrogen atom or a methylene group; wherein when C is a methylene group, R2 is a methylene linked to C forming a 6 membered N methyl-piperidine;
Z1, Z4 are both independently selected from a sulphur atom or a single bond forming a 5 membered ring; so that when Z1 is a sulphur atom, Z4 forms a single bond forming a 5 membered ring; or when Z4 is a sulphur atom, Z1 forms a single bond forming a 5 membered ring;
Z2 and Z3 are both carbon atoms; and
D1-D4 are carbon atoms.

3. The composition for use according to claim 1, wherein n = 0, A and B together represent a single bond so as to form a 6 membered ring, Y is a hydrogen atom, and X is a sulphur atom forming a 6 membered ring, wherein:
R2 is a methyl group and C is a hydrogen atom;
Z1, Z2 and Z4 are CH groups;
Z3 is a carbon atom, D4 is a Chlorine atom; and
D1, D2 and D3 are absent.

4. The composition for use according to claim 1, wherein n = 0, A and B together represent a single bond so as to form a 5 membered ring, Y is a hydrogen atom, and X is a single bond forming a 5 membered ring, wherein:
R2 is a methyl or methylene group;
C can be selected from a hydrogen atom or a methylene group; wherein when C is a methylene group, R2 is a methylene linked to C forming a 6 membered N methyl-piperidine;
Z1, Z4 are both independently selected from a sulphur atom or a single bond forming a 5 membered ring; so that when Z1 is a sulphur atom, Z4 forms a single bond forming a 5 membered ring; or when Z4 is a sulphur atom, Z1 forms a single bond forming a 5 membered ring;
Z2 and Z3 are both carbon atoms; and
D1-D4 are carbon atoms.

5. The composition for use according to claim 1, wherein n is 1, X is a sulphur atom, B and C together represent a single bond forming a 7 membered sulphur containing heterocyclic ring, A is absent and both R1 and R2 are both methylene groups linked together so as to form a 6 membered heterocyclic ring, Y is a Chlorine atom, Z1, Z2 and Z4 are CH groups, Z3 is a carbon atom, D1 is H, D2 and D3 are absent, and D4 is a fluorine atom.

6. The composition for use according to any of claims 1 to 5, wherein the leukaemia is selected from any of acute myeloid leukemia (AML), B and T acute lymphoblastic leukemias (B-ALL and T-ALL), or chronic myeloid leukemia (CML).

7. The composition for use according to claim 6, wherein the leukaemia is acute myeloid leukemia (AML).

8. The composition for use according to any of claims 1 to 7, wherein the compound of formula I is selected from any of formula: 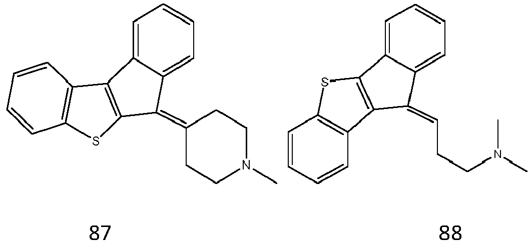 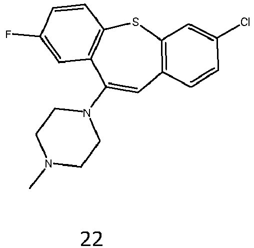 or any pharmaceutically acceptable salt or isomer thereof.

9. The composition for use according to the precedent claim, wherein the compound of formula I is: 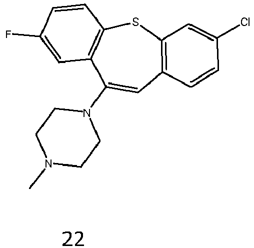 or any pharmaceutically acceptable salt or isomer thereof.

10. The composition for use according to claim 8, wherein the compound of formula I is selected from any of formula: 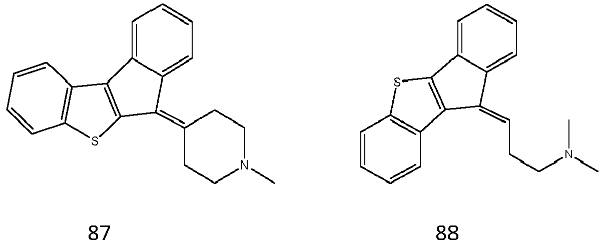 or any pharmaceutically acceptable salt or isomer thereof.

11. The composition for use according to claim 8, wherein the compound is: 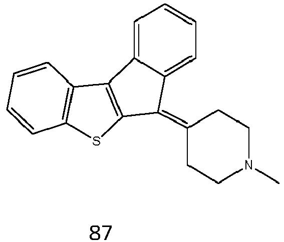 or any pharmaceutically acceptable salt or isomer thereof.

12. A pharmaceutical composition comprising a compound of formula I selected from any of formula: 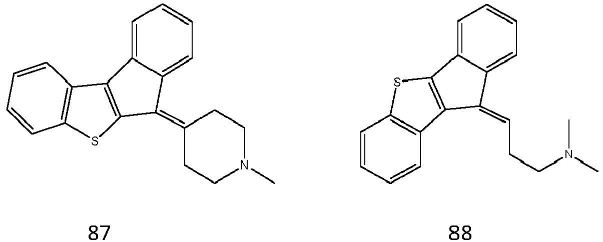 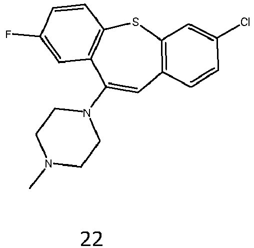 or any pharmaceutically acceptable salt or isomer thereof.

13. The composition according to the precedent claim, wherein the compound of formula I is: 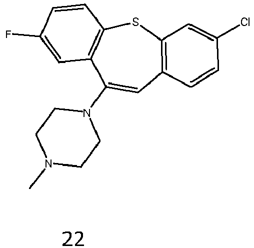 or any pharmaceutically acceptable salt or isomer thereof.

14. The composition according to claim 12, wherein the compound of formula I is 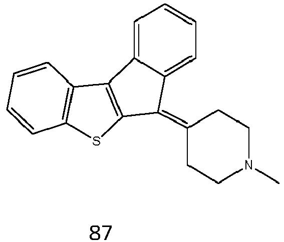 or any pharmaceutically acceptable salt or isomer thereof.

15. The composition according to claim 12, wherein the compound of formula I is 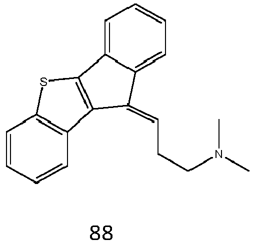 or any pharmaceutically acceptable salt or isomer thereof.
